## Europäisches Patentamt
## European Patent Office
## Office européen des brevets

(19)

(11) Veröffentlichungsnummer: **0 052 777**

**A1**

(12) **EUROPÄISCHE PATENTANMELDUNG**

(21) Anmeldenummer: **81108834.3**

(22) Anmeldetag: **23.10.81**

(51) Int. Cl.³: **C 07 C 175/00**
**C 07 C 31/22**

(30) Priorität: **20.11.80 CH 8594/80**

(43) Veröffentlichungstag der Anmeldung:
**02.06.82 Patentblatt 82/22**

(84) Benannte Vertragsstaaten:
**CH DE FR GB IT LI**

(71) Anmelder: **F. HOFFMANN-LA ROCHE & CO.
Aktiengesellschaft**

**CH-4002 Basel(CH)**

(72) Erfinder: **Rüegg, Rudolf, Dr.
Kreuzackerweg 10
CH-4103 Bottmingen(CH)**

(74) Vertreter: **Lederer, Franz, Dr. et al,
Patentanwälte Dr. Franz Lederer Dipl.-Ing. Reiner F.
Meyer-Roxlau Lucile-Grahn-Strasse 22
D-8000 München 80(DE)**

(54) **Verfahren zur Extraktion von beta-Carotin aus Algen.**

(57) Es wird ein Verfahren zur Gewinnung von β-Carotin bzw. von Glycerin aus gewissen Algen beschrieben. Hierbei wird das Algenmaterial zuerst mit Calciumhydroxid behandelt, dann wird das Gemisch filtriert und das β-Carotin aus dem Filterrückstand und das Glycerin aus dem Filtrat isoliert.

EP 0 052 777 A1

Croydon Printing Company Ltd.

Verfahren zur Extraktion von β-Carotin aus Algen

Die vorliegende Erfindung betrifft ein neues Verfahren zur Gewinnung von β-Carotin bzw. von Glycerin aus diese Substanzen enthaltenden Algen, insbesondere aus Dunaliella-Algen.

Es ist seit längerem bekannt, dass gewisse, insbesondere in stark salzhaltigen Gewässern vorkommende Algenarten, wie etwa diejenigen der Gattung Dunaliella, u.a. beträchtliche Mengen β-Carotin und Glycerin enthalten. Die Isolierung dieser Substanzen aus dem Algenmaterial hat jedoch bis jetzt erhebliche Schwierigkeiten bereitet, da letzteres entweder zuerst von dem Salzwasser in dem es gewachsen war abgetrennt werden musste, oder es mussten bei der Extraktion des Gemisches Salzwasser und Algen sehr grosse Mengen an organischen Extraktionsmitteln eingesetzt werden. Bei der Extraktion wurden zudem nicht nur β-Carotin, sondern auch das in den Algen enthaltene Chlorophyll extrahiert, was die Isolierung von reinem β-Carotin sehr erschwerte. Ausserdem musste das Gemisch während der Extraktion mit dem organischen Lösungsmittel mehr oder weniger stark geschüttelt werden, um die Algenzellen zum Platzen zu bringen.

Es bestand somit ein Bedürfnis nach einem Verfahren, mittels welchem es gelingen würde, sowohl β-Carotin als auch Glycerin auf einfache Art und Weise, mit befriedigender Ausbeuten und grösstmöglicher Reinheit aus den Algen zu extrahieren.

Dies ist nunmehr durch das erfindungsgemässe Verfahren gelungen. Dieses Verfahren ist dadurch gekennzeichnet, dass man rohes Algenmaterial mit Calciumhydroxid behandelt, dann das Gemisch filtriert, das β-Carotin aus dem Filterrückstand extrahiert und, gewünschtenfalls, das Glycerin aus dem Filtrat isoliert.

Das als Ausgangsmaterial verwendete Algenmaterial kann ungereinigt in dem erfindungsgemässen Verfahren verwendet werden, d.h. vor der Behandlung mit dem Calciumhydroxid brauchen die Algen nicht vollständig von vorhandenem Salzwasser abgetrennt zu werden. Die Behandlung des Algenmaterials mit Calciumhydroxid erfolgt zweckmässig unter Inertgasatmosphäre, z.B. unter Argon und bei einer Temperatur von etwa 50°C bis etwa 100°C, vorzugsweise bei Rückflusstemperatur des Gemisches. Das Erhitzen erfolgt zweckmässig während etwa 2-6 Stunden. Durch diese Behandlung wird das in den Algen vorhandene Chlorophyll in Calciumsalze übergeführt, welche in den im Nachfolgenden erwähnten Lösungsmittel für β-Carotin unlöslich sind.

Der Filterrückstand kann sofort in feuchtem Zustand, z.B. wasserfeucht oder mit Methanol oder Aethanol gewaschen, der Extraktion unterworfen werden. Vorzugsweise wird er jedoch vor der Extraktion vollständig getrocknet, beispielsweise im Vakuumtrockenschrank. Die Extraktion erfolgt in an sich bekannter Weise mit einem Lösungsmittel für β-Carotin, wie etwa halogenierte Kohlenwasserstoffe, z.B. Methylenchlorid, oder mit einem in Wasser nicht löslichen Lösungsmittel, z.B. aliphatische oder aromatische Kohlenwasserstoffe wie Hexan, Benzol, Toluol oder auch Petroläther und dergleichen.

Zur Isolierung des Glycerin aus dem Filtrat wird dieses zweckmässig zuerst mit Säure neutralisiert. Die Lösung wird dann eingeengt und der Rückstand durch Behandlung mit einem niederen Alkohol mit bis zu 5 Kohlenstoffatomen, vorzugsweise mit Isopropanol, gereinigt.

## Beispiel

500 g nasser Algenschlamm werden mit 75 g Calciumhydroxid unter Rühren und Argonbegasung während 2 Stunden auf ca. 100°C erwärmt. Nach dem Abkühlen auf ca. 50°C wird unter Zugabe von möglichst wenig Wasser abgenutscht und mit Wasser nachgewaschen. Man erhält ein schwach gelbgrün gefärbtes, klares Filtrat. Der Filterrückstand wird sodann vollständig getrocknet und mit Methylenchlorid im Soxhlett-Apparat extrahiert, bis der Extrakt praktisch farblos ist. Es bleiben ca. 110 g eines grüngefärbten Pulvers ungelöst zurück. Das Methylenchlorid wird nun abdestilliert und der halbfeste rote Rückstand mit 200 ml Hexan versetzt (eventuell Erwärmen und Umschwenken, sodass sich alles von der Kolbenwand löst) und über Nacht bei Raumtemperatur stehen gelassen. Hierauf wird abfiltriert und man erhält 1,9 g Rohcarotin (ca. 90%ig). Dieses Rohcarotin wird aus Methylenchlorid/Methanol umkristallisiert und man erhält 1,3 g 94,5%iges all-trans-β-Carotin: UV $\lambda_{max}$ 450 nm, $E_1^1$ 2560 (in Hexan).

Das vorhergehend erwähnte, schwach gelbgrün gefärbte Filtrat wird mit ca. 50%iger Schwefelsäure auf pH 6-7 neutralisiert. Die erhaltene Lösung wird - gewünschtenfalls nach Abfiltration des angefallenen Calciumsulfats - am Wasserstrahlvakuum eingedampft. Man erhält ca. 120 g eines gelblich gefärbten zähen Harzes. Dieses wird mit 300 ml Isopropanol einige Stunden geschüttelt, vom ungelösten Salz abfiltriert und eingedampft. Dieses Procedere wird nochmals mit 200 ml Isopropanol wiederholt. Man erhält 23 g schwach gelbgefärbtes Rohglycerin, das im Hochvakuum destilliert wird. Man erhält so 18,5 g reines Glycerin bei 0,03 mm Hg und 95 °C, $n_D^{20}$ 1,4740.

## Patentansprüche

1. Verfahren zur Gewinnung von β-Carotin bzw. von Glycerin aus diese Substanzen enthaltenden Algen, dadurch gekennzeichnet, dass man rohes Algenmaterial mit Calciumhydroxid behandelt, dann das Gemisch filtriert, das β-Carotin aus dem Filterrückstand extrahiert und, gewünschtenfalls, das Glycerin aus dem Filtrat isoliert.

2. Verfahren nach Anspruch 1, dadurch gekennzeichnet, dass man die Behandlung des Algenmaterials mit dem Calciumhydroxid bei einer Temperatur von etwa 50°C bis etwa 100°C, vorzugsweise bei Rückflusstemperatur des Gemisches, durchführt.

3. Verfahren nach Anspruch 1 oder 2, dadurch gekennzeichnet, dass man die Behandlung des Algenmaterials mit dem Calciumhydroxid während etwa 2-6 Stunden durchführt.

4. Verfahren nach einem der Ansprüche 1 bis 3, dadurch gekennzeichnet, dass man die Behandlung des rohen Algenmaterials mit dem Calciumhydroxid unter Inertgasatmosphäre durchführt.

5. Verfahren nach einem der Ansprüche 1 bis 4, dadurch gekennzeichnet, dass man den Filterrückstand vor der Extraktion vollständig trocknet.

6. Verfahren nach einem der Ansprüche 1 bis 5, dadurch gekennzeichnet, dass man die Extraktion mit Methylenchlorid, Hexan oder hochsiedendem Petroläther durchführt.

***

Europäisches
Patentamt

**EUROPÄISCHER RECHERCHENBERICHT**

| EINSCHLÄGIGE DOKUMENTE | | | KLASSIFIKATION DER ANMELDUNG (Int. Cl.³) |
|---|---|---|---|
| **Kategorie** | **Kennzeichnung des Dokuments mit Angabe, soweit erforderlich, der maßgeblichen Teile** | **betrifft Anspruch** | |
| A | US - A - 4 115 949 (M. AVRON et al.) <br> * Spalte 5, Zeilen 17 bis 49 * <br> --- | 1,5,6 | C 07 C 175/00 <br> C 07 C 31/22 |
| A | US - A - 4 199 895 (M. AVRON et al.) <br> * Spalte 4, Zeilen 24 bis 45 * <br> --- | 1,6 | |
| A | DE - B - 1 203 222 (MAGGI AG) <br> * vollständiges Dokument * <br> --- | | |
| A | US - A - 1 786 095 (K. TAKAHASHI <br> et al.) <br> * Spalte 1, Zeilen 1 bis 18; <br> Beispiele 1, 2 * <br> --- | | **RECHERCHIERTE SACHGEBIETE (Int. Cl.³)** <br> C 07 C 31/22 <br> C 07 C 175/00 |
| A | ULLMANNS ENCYKLOPÄDIE DER TECHNISCHEN <br> CHEMIE, <br> 3. Auflage, Band 7, <br> 1956, URBAN & SCHWARZENBERG, <br> München-Berlin <br> Seiten 93 bis 101 <br> * Seite 97, Zeilen 1 bis 10; <br> Seite 98, Zeilen 21 bis 40 * <br> ---- | | C 12 N 1/12 <br> C 12 P 1/00 <br> C 12 P 5/00 <br> C 12 P 7/00 <br> C 12 P 23/00 <br> C 12 R 1/89 |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| Berlin | 03-02-1982 | BERTRAM |

EPA form 1503.1  08.78